# EUROPEAN PATENT APPLICATION

(11) **EP 4 480 408 A1**
(43) Date of publication of application: **25.12.2024**
(21) Application number: 23180348.7
(22) Date of filing: 20.06.2023
(51) Int. Cl.: A61B 5/145, A61B 5/1468, A61B 5/263

(54) **ELECTROCHEMICAL DEVICE COMPRISING ELECTRODE COATED WITH CROSSLINKED POLYMERIC COATING**

(71) Applicant: Universiteit Antwerpen, 2000 Antwerpen (BE)
(72) Inventor: Parrilla Pons, Marc, 2020 Antwerpen (BE); De Wael, Karolien, 2020 Antwerpen (BE)
(74) Representative: De Clercq & Partners

(57) **Abstract**

The present application provides a sensor device and related methods for electrochemically measuring a substance in a fluid, particularly a bodily fluid, wherein said sensor device comprises one or more electrodes, wherein said one or more electrodes comprise at least a working electrode, a reference electrode and/or a counter electrode, wherein the surface of at least the working electrode is coated with a crosslinked polymer, such as a crosslinked chitosan, a crosslinked PEI, a crosslinked polylysine, and the like. In particular embodiments, the sensor device may comprise a microneedle array comprising a plurality of microneedle electrodes, wherein at least the working microneedle electrode is coated with a crosslinked polymer. The present application further provides a system and related methods for the monitoring of a first substance and delivery of a second substance in the dermal interstitial fluid of a subject, wherein the system comprises the sensor device and a drug delivery system.

## Description

### Field of the invention

The present invention is situated in the field of electrochemical devices, systems and related methods for analyte monitoring in a fluid. More in particular, the present invention provides electrochemical devices, systems and methods allowing for the continuous determination of the concentration of various analytes or substances in a sample, such as a bodily fluid, even more in particular in a bodily fluid within a body.

### Background of the invention

Electrochemical devices for monitoring of analytes, such as pharmaceutical compounds or metabolites thereof in fluids, particularly biofluids, are known. However, biofouling or passivation of the surface of the electrode(s) of the electrochemical device due to the adsorption of biomolecules (e.g. protein and lipids) hinders the proper detection/monitoring. Particularly, when passivation occurs, the diffusion of the analyte towards the electrode's surface is blocked, and thus, there is a loss of the performance of the sensor as the analytical signal is reduced. Therefore, there is a need for a method to avoid this passivation while allowing the diffusion of the analyte of interest. Several coatings have been used for this purpose. However, the coating normally hinders the diffusion toward the electrode thus limiting highly sensitive detection. Ideally, a coating that avoids biofouling while allowing the fast diffusion of the analyte toward an electrode's surface for sensitive detection is desired.

Wearable electrochemical devices, particularly wearable microneedle (MN) based electrochemical devices, have revolutionized how chemical data from patients is obtained and analyzed. Wearable MN-based electrochemical sensors have been produced under different configurations: (i) a conductive solid MN array can be functionalized (e.g. with nanomaterials, mediator, enzyme); (ii) a hollow microneedle array (HMA) can be filled with conductive pastes to attain an electrochemical cell; (iii) a HMA can be used for the extraction of interstitial fluid toward an electrochemical sensor at the back side of the HMA; or a miniaturized electrode/sensor is inserted in a polymeric MN array with the capability to uptake interstitial fluid.

More specifically, transdermal diagnostics with MNs represents an appealing technology to monitor analytes in interstitial fluid due to its minimal invasiveness, the ability to monitor analytes in real time, and the simplicity and availability of the matrix in comparison to blood. Hence, there is a rising interest in drug monitoring via interstitial fluid due to the opportunities that electrochemical sensors offer in drug detection, particularly fitting the therapeutic range, sensitivity and limit of detection (LOD). Three strategies are mainly approached for drug monitoring using MN arrays: (i) the use of direct electrochemical oxidation at the surface of the HMA electrode; (ii) the functionalization of solid MNs with aptamers for different antibiotics; and (iii) the use of swellable MNs mounted on interdigitated electrodes. Accurate monitoring of the drug or its metabolite also allows closed loop systems comprising wearable drug delivery devices. However, many challenges remain in the engineering of the MN sensor, in improving the stability for continuous operation, in improving the sensitivity and limit of detection, and the ability to reduce biofouling for undiluted analysis in biofluids.

Therefore, there is a need in the art for improved electrochemical devices, including but not limited to microneedle based electrochemical devices, for analyte monitoring.

### Summary of the invention

The inventors have developed an improved electrochemical sensor wherein an electrode, particularly at least the working electrode is coated with a crosslinked polymer, such as a crosslinked chitosan. Advantageously, this coating conferred a synergic effect of anti-biofouling and preconcentration capabilities for the electrochemical sensor. In a particular embodiment, the electrochemical sensor according to the present application is a microneedle based electrochemical sensor for the transdermal monitoring of an analyte, such as methotrexate (MTX), wherein the microneedle based electrochemical sensor is functionalized by coating an electrode with the crosslinked polymer coating. The thus functionalized microneedle based electrochemical sensor exhibited an outstanding performance regarding the linear detection range, the reversibility and long-term stability of the sensor. Furthermore, under an *ex vivo* configuration in porcine skin, the electrochemical sensor demonstrated the ability to transdermally monitor decreasing concentrations of an analyte, such as MTX, thus allowing to observe the clearance of a drug, such as MTX, in patients under MTX treatment. The electrochemical sensor could further be developed in an on-demand iontophoretic system, contributing toward a closed-loop sensing and delivery system for the self-sufficient management of therapy, such as MTX therapy in cancer patients.

A first aspect of the present application provides a sensor device for electrochemically measuring a substance in a sample, such as a bodily fluid, said sensor device comprising one or more electrodes, wherein said one or more electrodes comprise at least a working electrode, a reference electrode and/or a counter electrode, wherein the surface of at least the working electrode is coated with a coating, characterized in that the coating comprises a crosslinked polymer, preferably a crosslinked polymer carrying a positively or negatively charged ionizable functional group. In particular embodiments, the crosslinked polymer is a crosslinked chitosan, a crosslinked chitosan derivative, a crosslinked polyethyleneimine, a crosslinked polylysine, a crosslinked polyallylamine, a crosslinked poly(diallyl dimethyl ammonium chloride), a crosslinked polyvinyl alcohol, or a mix or copolymer thereof. The coating may have a thickness ranging between 0.1 and 15 µm, particularly between 0.5 and 10 µm. More in particular, the crosslinked polymer has been or is obtained by reacting a polymer and a crosslinking agent at a polymer to crosslinking agent ratio of 0.01 to 20 by weight, particularly a polymer to crosslinking agent ratio of 0.05 to 15. The coating may further comprise nanomaterials or nanocomposites to increase the electroactive surface area of the electrode and to attain an increment of the sensitivity and a decrease in the limit of detection of the substance.

The substance may be a small molecule, such as a pharmaceutical compound or a metabolite thereof.

In certain embodiments, the sensor device is an electrochemical point-of-care device.

In further particular embodiments, a sensor device is provided, particularly a microneedle-based sensor device, for measuring the substance in the dermal interstitial fluid of a subject, wherein the device comprises:
- a substrate configured for attachment to the skin of the subject; and
- a microneedle array comprising a plurality of microneedles, coupled to the substrate, wherein said plurality of microneedles comprises a microneedle working electrode made up of a first microneedle or a first series of microneedles, said microneedle(s) comprising a conductive material, and further comprising a microneedle reference electrode made up of a second microneedle or a second series of microneedles, said microneedle(s) comprising a conductive material, and/or a microneedle counter electrode made up of a third microneedle or a third series of microneedles, said microneedle(s) comprising a conductive material, wherein at least the microneedle working electrode is coated with the coating comprising the crosslinked polymer, particularly a crosslinked polymer carrying a positively or negatively charged ionizable functional group, more particularly is coated with crosslinked chitosan, crosslinked chitosan derivative or a crosslinked polyethyleneimine, crosslinked polylysine, crosslinked polyallyl amine, crosslinked poly(diallyl dimethyl ammonium chloride), a crosslinked polyvinyl alcohol, or a mix or copolymer thereof. In particular embodiments, said plurality of microneedles comprises a plurality of microneedles with a hollow center, wherein the hollow center comprises a conductive material, such as a paste made of a conductive material for the working and counter electrode, and/or a silver/silver chloride paste for the reference electrode; or said plurality of microneedles comprises a plurality of conductive solid microneedles. Said sensor device may be a voltammetric or an amperometric sensor device.

In certain embodiments, the sensor device further comprises one or more of (i) a potentiostat, coupled to the one or more electrodes via a conductive connector; (ii) a processing unit, coupled to the potentiostat, for measuring electric signals via the electrodes and analyzing the electric signals; (iii) a memory unit, coupled to the processing unit, for storing data regarding the measured electric signals and/or the analysis thereof, and comprising instructions for the processing unit; (iv) a transmitter, coupled to the processing unit, for transmitting a signal to an external device, system or network, for instance wherein said external device, system or network includes a display or a user control for the sensor device; (v) optionally, a plug-in connector as an interface between a disposable microneedle array and an electronics unit; and/or (vi) optionally, means for the sustained attachment and/or proper insertion of the sensor device to the skin of the subject. More in particular, the instructions for the processing unit include the steps of (i) applying a specific voltage or scanning/sweeping voltage in different ramp forms, such as linear sweep, a square-wave form, a differential-pulse form, a triangular wave form, to the microneedle array based electrochemical cell, (ii) measuring an electrical current in response to the applied voltage, and (iii) determining the concentration of the substance in the dermal interstitial fluid of the subject using the measured current.

A further related aspect of the present application provides a system, particularly a closed loop system for the monitoring of a first substance and delivery of a second substance, particularly in the dermal interstitial fluid of a subject, wherein optionally said first and second substance are identical, the system comprising:
- a sensor device, particularly a microneedle-based sensor device according to the present application, for electrochemically measuring a substance according to the present application, wherein said substance is the first substance;
- a first and second electrode, such as wherein the first electrode is the cathode and the second electrode is the anode;
- a drug delivery device comprising the second substance, wherein the drug delivery device is operably connected to the first electrode and is configured to release the second substance into the dermal interstitial fluid of the subject upon applying an electrical current between the first electrode and the second electrode; and
- a power supply connected to the first and second electrodes.

In certain embodiments, the system further comprises one or more of:
- a processing unit coupled to the sensor device and the power supply, comprising instructions for controlling the monitoring of the first substance or a metabolite thereof in the dermal interstitial fluid and for controlling the administration of the second substance from the drug delivery device, particularly wherein the processing unit correlates a signal generated by the sensor device to the concentration of the first substance in the dermal interstitial fluid, compares this concentration to a threshold value, and activates the power supply if the concentration of the first substance is below the threshold value;
- a memory unit, coupled to the processing unit, for storing data regarding the measured signals and/or the analysis thereof, and/or comprising instructions for the processing unit;
- an algorithm for sensing and actuating that calculates the amount of the second substance that needs to be delivered according to the concentration in the interstitial fluid and is extrapolated to the current and time needed in the drug delivery device;
- a transmitter, coupled to the processing unit, for transmitting a signal to an external device, system or network; and/or
- means for the sustained attachment and/or proper insertion of the system to the skin of the subject.

In particular embodiments, the sensor device or the system according to the present application is a wearable device.

A further related aspect of the present application provides the use of a crosslinked polymer, particularly a crosslinked polymer carrying a positively or negatively charged ionizable functional group, for coating the surface of at least a working electrode of a sensor device for electrochemically measuring a substance in a bodily fluid, more particularly wherein said crosslinked polymer is crosslinked chitosan, crosslinked chitosan derivative, crosslinked polyethyleneimine, crosslinked polylysine, crosslinked polyallyl amine, crosslinked poly(diallyl dimethyl ammonium chloride), crosslinked polyvinyl alcohol or a mix or copolymer thereof, optionally wherein the coating further comprises conductive nanomaterials or nanocomposites.

A further related aspect provides a method for measuring a substance in a bodily fluid of a subject, particularly the dermal interstitial fluid of a subject, in particular wherein the substance is a pharmaceutical compound or a metabolite thereof, comprising the steps of:
(a) attaching a sensor device or a system according to the present application to the skin of the subject, thereby contacting the microneedle array with the substance in the bodily fluid of the subject; and
(b) measuring an electrical signal via the one or more electrodes, wherein the measured electrical signal is dependent on the presence, absence or concentration of the substance in the bodily fluid.

In particular embodiments, step (b) includes (i) applying a fix voltage and measuring the electrical signal output or applying a voltage through a voltage window from -0.9V to 1.2V and/or from 0V to -0.9V, such as a linear sweep, a square-wave form, a differential-pulse form, a triangular wave form and measuring the electrical signal, to the microneedle array, (ii) measuring an electrical current in response to the applied voltage, and (iii) determining the presence, absence or concentration of the substance in the bodily fluid of the subject using the measured current or total charge.

In particular embodiments, step (a) comprises attaching a system according to the present application to the skin of the subject, wherein the drug delivery device of the system comprises a second substance and the method further comprises the step of (c) providing the second substance to the subject by actuating the drug delivery device, when the measured signal indicates the absence or a too low concentration of the first substance in the subject.

In particular embodiments, the sensor device or the system according to the present application is continuously attached to the skin of the subject for a time period of a plurality of days and wherein step (b) is performed repeatedly during said time period, for determining the evolution of the presence, absence or concentration of the substance over time.

### Description of the Figures

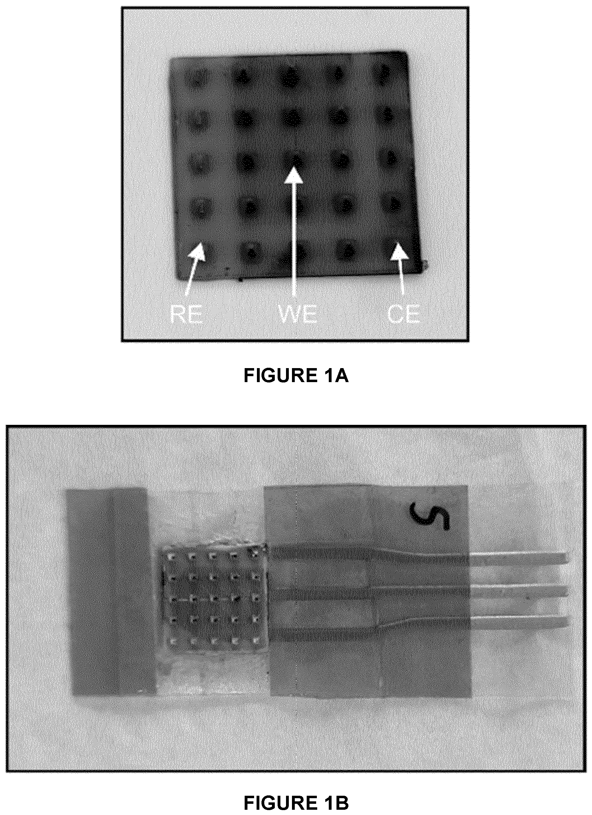

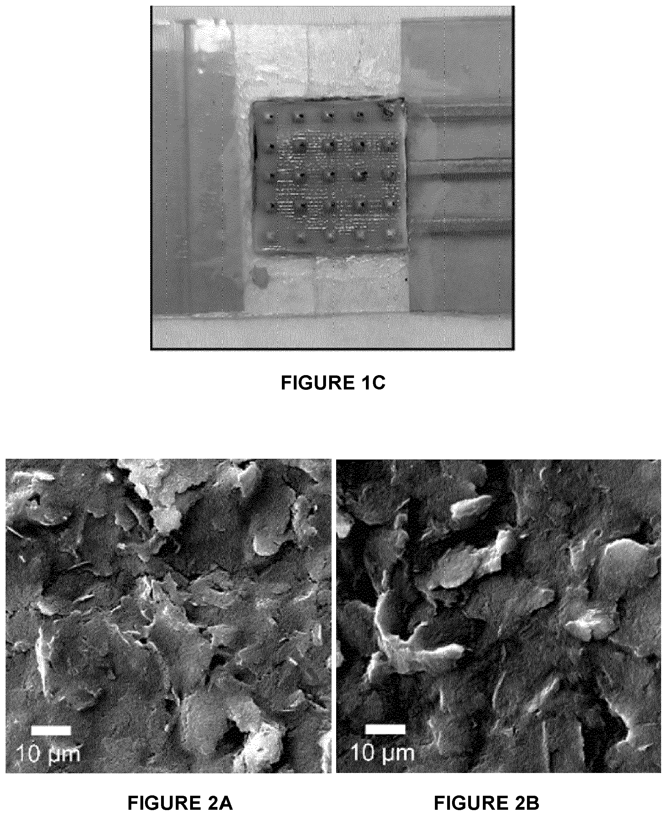

Figure 1 shows the fabrication of an embodiment of a microneedle-based sensor device. (A) front image of the sensor device with a three-electrode configuration, after ink filing and curing; (B) front image of the sensor device attached to a screen-printed connector; (C) image of the sensor device with a crosslinked chitosan layer deposited on the working electrode. CE = counter electrode; RE = reference electrode; WE = working electrode.

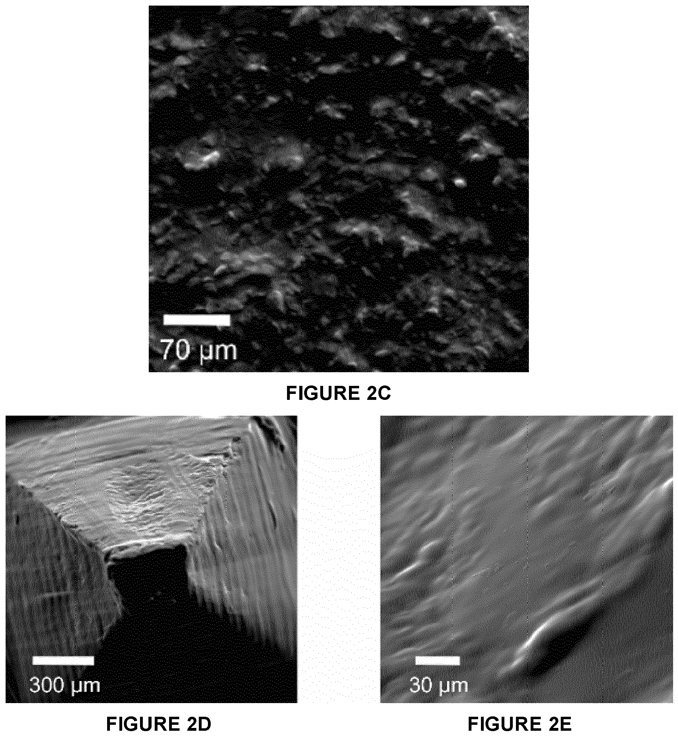

Figure 2 shows the SEM images for the evaluation of the deposited chitosan layer. (A) the surface of a bare working electrode (SPE); (B) the surface of a screen-printed electrode coated with a chitosan layer; (C) the surface of a screen-printed electrode coated with a crosslinked chitosan layer; (D), (E). SEM images of the hollow microneedles array, modified with a crosslinked chitosan layer.

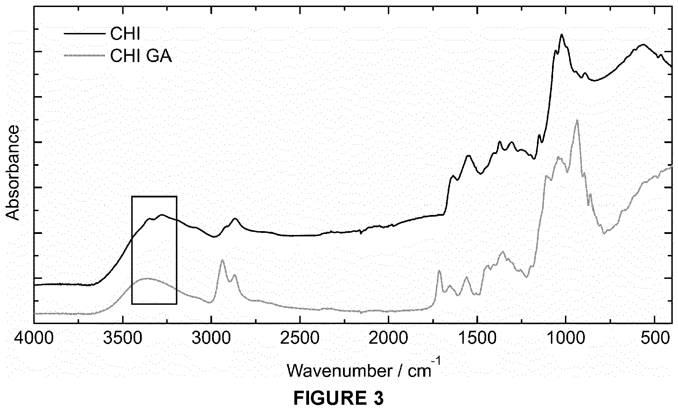

Figure 3 shows the chemical analysis of the deposited chitosan coatings at the surface of the electrode by ATR-FTIR characterization. Top spectrum: CHI/non-crosslinked chitosan; Bottom spectrum: CHI GA/crosslinked chitosan.

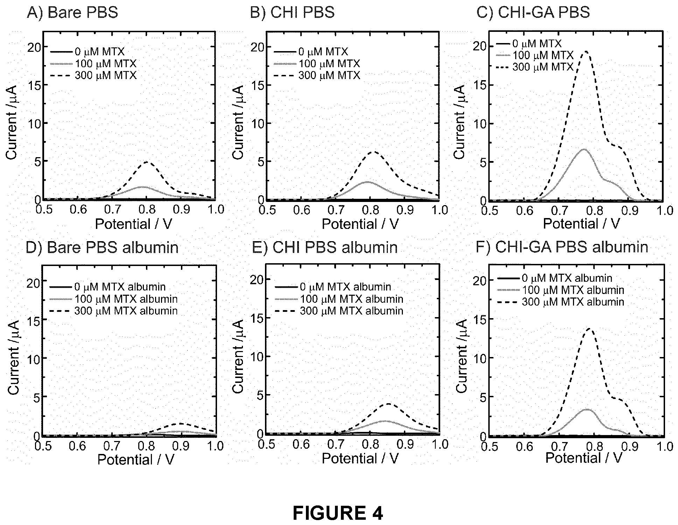

Figure 4 shows the electrochemical characterization of the methotrexate (MTX) oxidation on (A) bare SPEs; (B) non-crosslinked chitosan (CHI) coated SPEs; and (C) crosslinked chitosan (CHI/GA) coated SPEs in PBS pH 7.4, and (D) bare SPEs; (E) CHI coated SPEs; and (F) crosslinked CHI/GA coated SPEs in PBS pH 7.4 with 20.6 g/L of BSA. Accumulation time of 10 min.

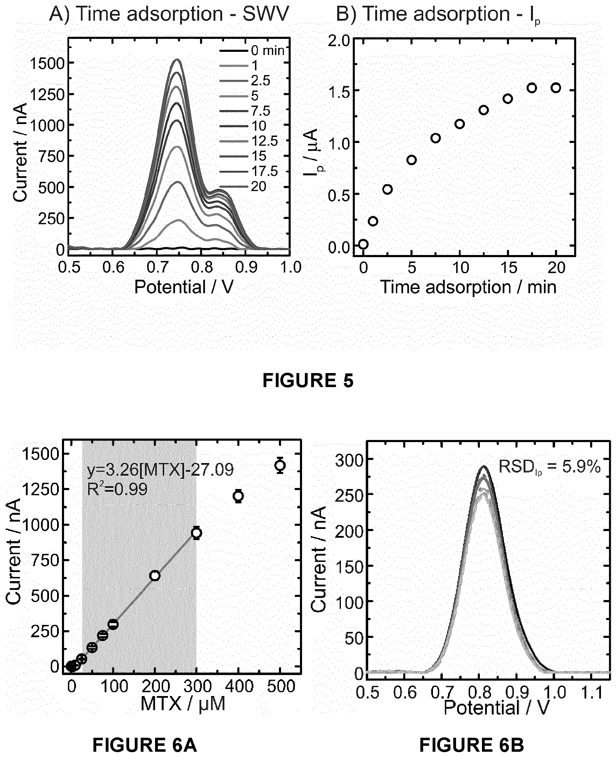

Figure 5 represents the results of an adsorption time study on a modified MN sensor with the crosslinked chitosan (CHI/GA) coating. (A) SWV of 500 µM MTX in PBS pH 7.4 upon increasing incubation time; and (B) peak current obtained from the left peak at 0.75 V vs. time of adsorption. SWV=square wave voltammetry.

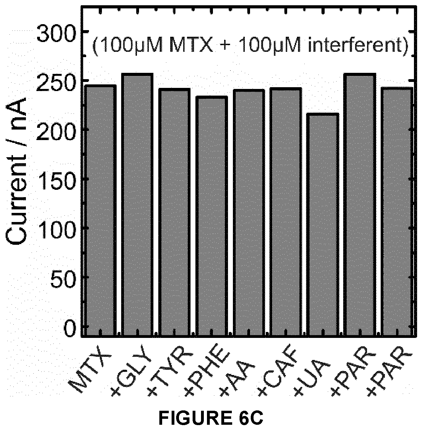

Figure 6 represents the *in vitro* analytical characterization of a reference MN-based electrochemical sensor without a polymeric coating. (A) calibration plot using the peak current of SWV of MTX analysis (N=3); (B) repeatability study for 100 µM MTX (N=10); (C) peak current obtained from interferent study with 100 µM MTX mixtures and 100 µM of interferents. AA=ascorbic acid; CAF=caffeine; Gly=glycine; PAR=paracetamol; PHE=phenylalanine; TYR=Tyrosine; UA=uric acid.

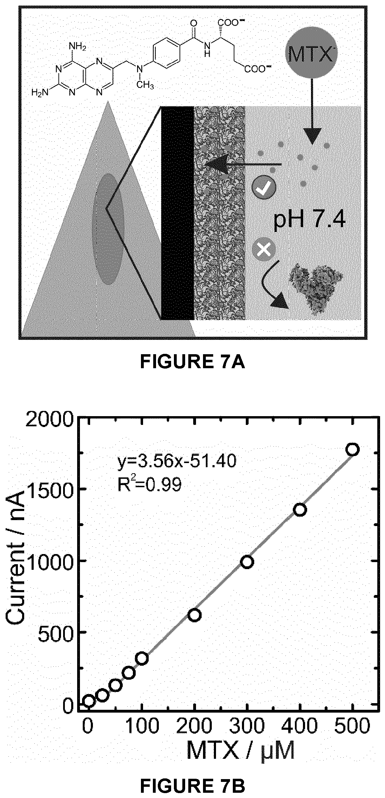

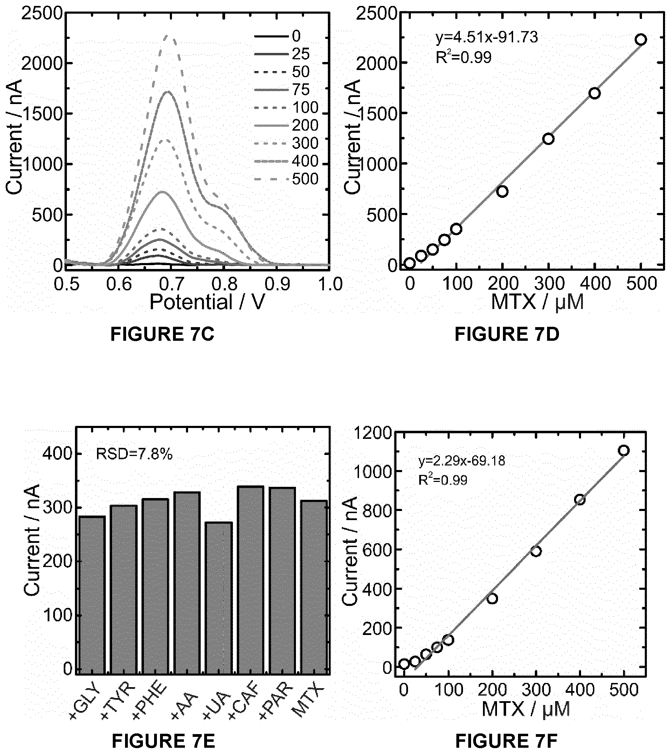

Figure 7 represents the *in vitro* analytical characterization of the functionalized MN-based electrochemical sensor with the crosslinked chitosan (CHI/GA) coating in PBS pH 7.4. (A) Diagram of the anti-biofouling mechanism employing the crosslinked CHI/GA coating. (B) calibration curve based on SWVs of increasing concentrations of MTX at 21 C°; (C) SWVs of increasing concentrations of MTX at 34 C° and (D) corresponding calibration curve. (E) Peak current from the anodic event from SWV from the selectivity test by using 100 µM MTX and interferent. All experiments were performed in PBS pH 7.4. Test under biofouling agent (20.6 g L⁻¹ of BSA): (F) calibration curve based on SWVs of increasing concentrations of MTX at 21°C.

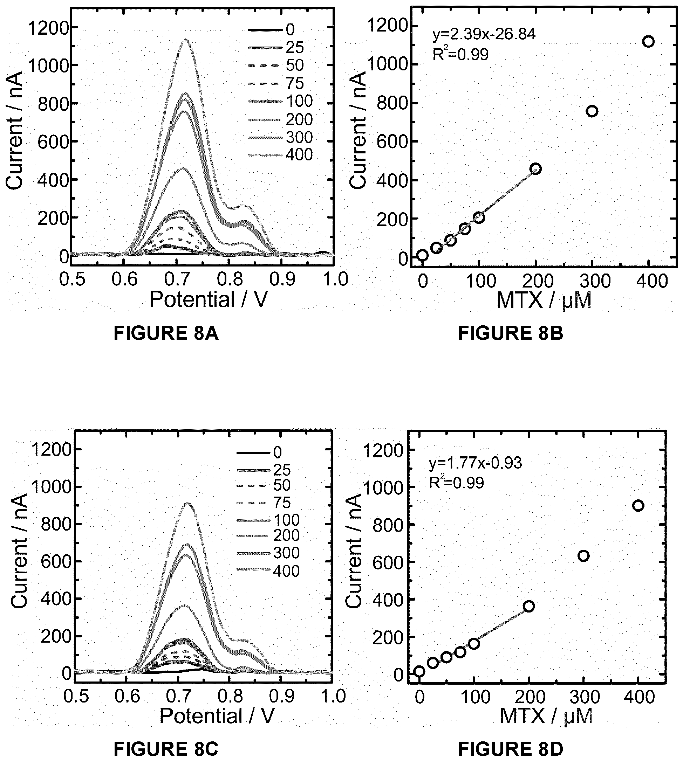

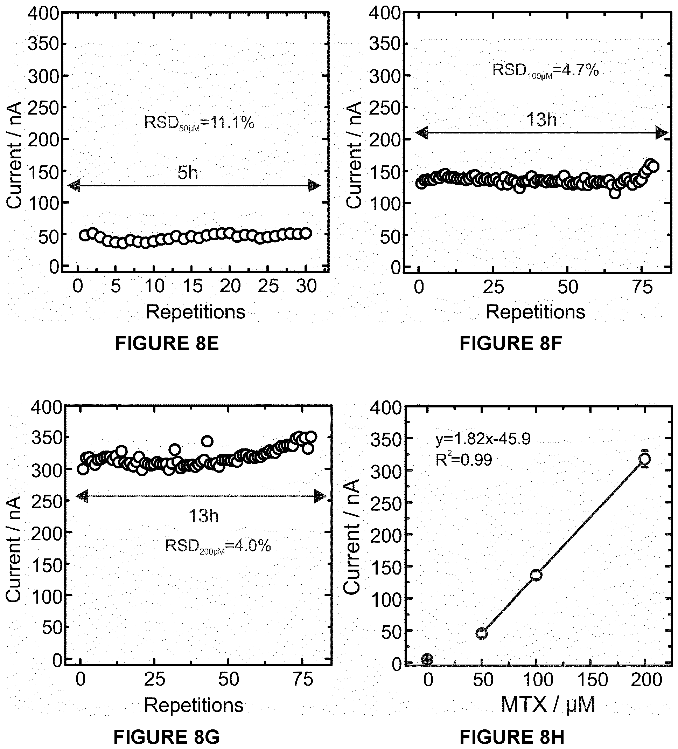

Figure 8 represents the analytical performance of the crosslinked chitosan coated MN sensor in artificial interstitial fluid (AISF): (A) SWVs and (B) corresponding calibration curve in AISF. (C) SWV and (D) corresponding calibration curves in AISF containing BSA (20.6 g L⁻¹). Stability test in AISF containing BSA for (E) 50 µM, (F) 100 µM, (G) 200 µM and (H) corresponding calibration curve using the peak current (Iₚ) values from the stability tests. The interval time between electrochemical experiments was set to 10 min.

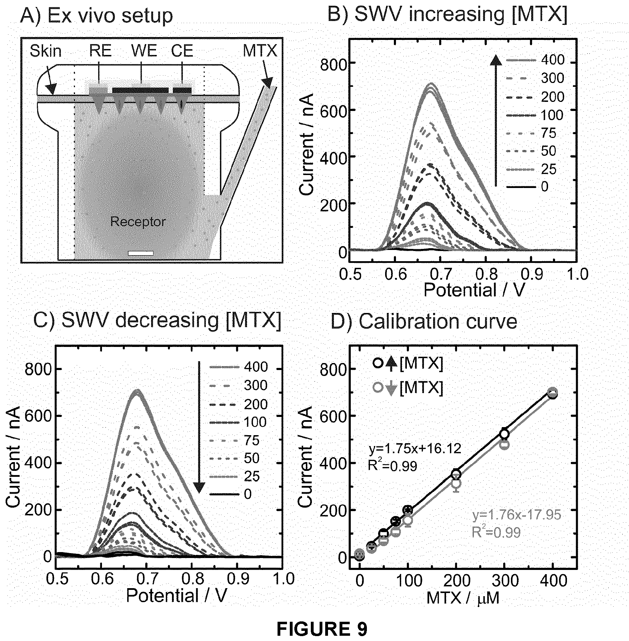

Figure 9 represents the ex *vivo* analytical characterization of the crosslinked chitosan coated MN sensor in a Franz diffusion cell using porcine skin. (A) Schematics of the ex vivo setup. (B) SWVs of increasing concentrations of MTX in the cell. (C) SWVs of decreasing concentrations of MTX in the cell. (D) Corresponding calibration curves for increasing and decreasing MTX concentration. The interval time between electrochemical experiments was set to 10 min.

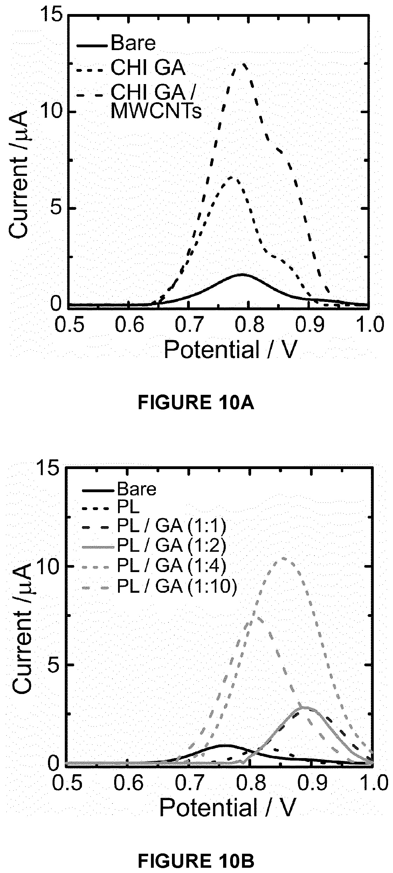

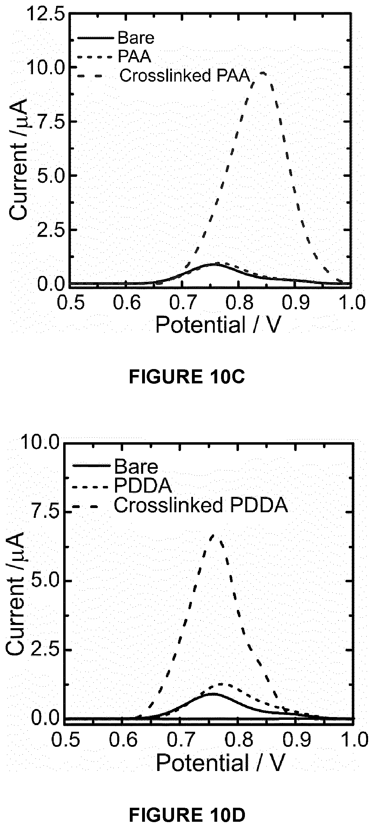

Figure 10 represents the electrochemical characterization of the methotrexate (MTX) oxidation in PBS pH 7.4 of SPEs coated with different coatings. (A) SPE coated with crosslinked chitosan and carbon nanotubes is compared with an uncoated SPE and an SPE coated with crosslinked chitosan without carbon nanotubes; (B) SPE coated with non-crosslinked (PL) and crosslinked polylysine (PL/GA) with PUGA ratio of 1:1; 1:2; 1:4 and 1:10); (C) SPE coated with non-crosslinked and crosslinked polyallyl amine (PAA); and (D) SPE coated with non-crosslinked and crosslinked poly(allyl dimethylammonium chloride). Accumulation time of 10 min.

### Detailed description of invention

Before the present system and method of the invention are described, it is noted that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or openended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g*., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

It is to be understood that other embodiments may be utilised, and structural or logical changes may be made without departing from the scope of the present invention. The following detailed description, therefore, is not to be taken in a limiting sense, and the scope of the present invention is defined by the appended claims.

The terms "determining," "measuring," "evaluating," "assessing," "assaying," and "analyzing" are often used interchangeably herein to refer to forms of measurement. These terms include determining if an element is present or not (for example, detection), but can also include quantitative and/or qualitative determinations, or relative or absolute determinations.

The inventors have developed an improved electrochemical sensor device comprising at least one electrode coated with a crosslinked polymer. The electrochemical sensor device is particularly suitable for measuring a substance or analyte in a bodily fluid, and exhibits an improved sensitivity, including to detect fluctuations in the concentration of the analyte of interest, an improved stability and an outstanding resistance to biofouling, compared to an electrochemical sensor with uncoated electrodes or with electrodes coated with non-crosslinked polymers.

A first aspect of the present application provides a sensor device, particularly for electrochemically measuring a substance in a fluid, such as a bodily fluid, said sensor device comprising one or more electrodes, wherein said one or more electrodes comprise at least a working electrode, and a reference electrode and/or a counter electrode, wherein the surface of at least the working electrode is coated with a coating, characterized in that the coating comprises a crosslinked polymer.

An "electrode" typically refers to a conductive part of the electrochemical sensor device which can contact the fluid sample, directly or via at least one semipermeable layer, such as the coating. The sensor device as envisaged may comprise a working electrode, a counter electrode, and a reference electrode. The "working electrode" refers to the electrode where oxidative and/or reductive processes, and the corresponding transfers of electrons, occur at its surface, particularly when a potential is applied to the one or more electrodes, particularly between the working electrode and the reference electrode and/or the counter electrode. A "counter electrode" refers to an electrode that ensures the proper current flow between the working and the counter electrodes. The "reference electrode" refers to an electrode that has a stable and well-known electrode potential thus providing the correct potential difference between the reference electrode and the working electrode. For example, the reference electrode can be silver/silver chloride (Ag/AgCI) electrode or saturated calomel electrode (SCE). An electrical potential may be applied between the working electrode and the counter electrode, and the current generated thereby can be measured between the working electrode and the counter electrode. In some embodiments, the reference electrode and the counter electrode may be the same electrode. In certain embodiments, the electrodes described herein comprise screen-printed electrodes (SPEs). As used herein, the term "screen printed electrode" refers to an electrochemical device that is manufactured by printing inks/pastes on substrates. In embodiments the inks/pastes can comprise carbon, silver, gold, platinum, or a combination thereof. In embodiments, the substrates can comprise plastic, paper, textile or ceramic substrates.

Advantageously, the coating of at least the working electrode by a crosslinked polymer exhibits a synergic effect for the detection of a substance of interest. The crosslinked polymer coating acts as a barrier for macromolecules, such as proteins, present in the bodily fluid, and prevents their adsorption to the electrode's surface, and thus provides anti-biofouling properties to the sensor device in general, and to the working electrode in particular. The crosslinked polymer coating further promotes the concentration and adsorption of the substance close to the electrode's surface, thereby catalyzing the electrochemical reaction and promoting the separation of the substance from other components in the fluid, thus increasing the sensitivity of the sensor. In certain embodiments, the coating may also result in the decrease of the oxidation potential.

It is understood that a crosslinked polymer is the product of the reaction between a polymer and a crosslinking agent.

The crosslinked polymer may be nonionic, positively or negatively charged. The polymer present in the crosslinked polymer may be a mixture of two or more polymers. The polymer present in the crosslinked polymer may be a homopolymer, i.e. a polymer derived from one species of monomer, or a copolymer, i.e. a polymer derived from more than one species of monomer, which may be randomly or non-randomly distributed in the polymer. The polymer present in the crosslinked polymer may be linear or branched.

In particular embodiments, the crosslinked polymer is a crosslinked chitosan or a crosslinked chitosan derivative, a crosslinked polyethyleneimine, a crosslinked polylysine, a crosslinked polyallylamine, a crosslinked poly(diallyl dimethylammonium chloride), a crosslinked polyvinylalcohol, or a mix or copolymer thereof. In certain embodiments, the crosslinked polymer comprises or is derived from a polymer carrying a positively or negatively charged ionizable functional group. An "ionizable functional group" as envisaged herein is a specific chemical moiety or a part of a molecule that can either gain or lose protons in a solution, depending on the solution's pH, thereby becoming positively charged (e.g. in the case of an amine group), or negatively charged (e.g. in the case of a carboxyl group). More in particular, the crosslinked polymer comprises or is derived from a polymer carrying a positively charged ionizable functional group, including but not limited to chitosan, polyethyleneimine, polylysine, polyallyl amine or poly(diallyl dimethyl ammonium chloride). Crosslinked polymers carrying a charged ionizable functional group may promote the concentration and adsorption of an oppositely charged substance. Accordingly, in particularly preferred embodiments, the crosslinked polymer is crosslinked chitosan or crosslinked chitosan derivative, crosslinked polyethyleneimine, crosslinked polylysine, crosslinked polyallyl amine, or crosslinked poly(diallyl dimethyl ammonium chloride), or a mix or copolymer thereof.

Chitosan, herein also abbreviated as CHI, is a natural polysaccharide, typically obtained by (partial) deacetylation of chitin. Chitosan is a linear polysaccharide composed of randomly distributed β-(1→4)-linked D-glucosamine (deacetylated unit) and N-acetyl-D-glucosamine (acetylated unit). The amino group in chitosan is significantly protonated in neutral solution. Chitosan crosslinking is known to the skilled person. Commonly used crosslinking agents for crosslinking chitosan include glutaraldehyde (and other dialdehydes), which reacts with the amino groups of chitosan; epoxy compounds, such as epichlorohydrin, which reacts with the hydroxyl and amino groups of chitosan; polyols, such as glycerol, ethylene glycol or polyethylene glycol, or poly- or dicarboxylic acids. Chitosan derivatives include for instance the modification of chitosan by acylation, carboxylation, alkylation, and quaternization. In particular embodiments, the chitosan as envisaged herein is at least 75% deacetylated, particularly is between 75% and 95% deacetylated.

Polyethyleneimine (herein abbreviated as PEI) is a polymer with repeating units of -[CH₂CH₂NH]-. PEI may be linear with chemical formula (C₂H₅N)ₙ, wherein the amine functional groups are secondary amines, or branched, comprising primary, secondary and tertiary amine groups. PEI has a polycationic character due to protonation of the amine group. Commonly used crosslinking agents for crosslinking PEI include glutaraldehyde and other dialdehydes; epoxy compounds, such as epichlorohydrin, or isocyanates.

Polylysine (herein also abbreviated as PL) is a homopolypeptide consisting of repeating lysine amino acid units, which may be linked via the amino group at the α carbon or at the ε carbon of the lysine molecule. At pH 7 it contains a positively charged amino group. Crosslinking agents for polylysine are known, and include glutaraldehyde.

Polyallylamine, also known as polyallylamine hydrochloride and herein abbreviated as PAA, is a cationic polymer prepared by the polymerization of allylamine (CH₂CHNH₂). Crosslinking agents for polyallylamine are known, and include glutaraldehyde.

Poly(diallyl dimethylammonium chloride), herein also abbreviated as PDDA, is a homopolymer of diallyldimethylammonium chloride. Crosslinking agents for PDDA are known, and include glutaraldehyde.

Polyvinyl alcohol (herein also abbreviated as PVA) is a synthetic polymer according to the chemical formula (C₂H₄O)ₙ or [CH₂CH(OH)]ₙ. PVA crosslinking is known in the art and includes chemical crosslinking with crosslinking agents, such as glutaraldehyde. PVA may also be crosslinked by esterification reactions, by reacting the PVA with a carboxylic acid or acid derivative.

It is understood that, particularly prior to crosslinking, the polymer may have a broad molecular weight range. More in particular, prior to crosslinking, the chitosan may be low-molecular-weight chitosan, with an average molecular mass of less than about 100 kDa; medium-molecular-weight chitosan, with an average molecular mass between about 100 kDa and about 700 kDa; and high-molecular weight chitosan, with molecular mass above about 700 kDa. Prior to crosslinking, linear or branched PEI may have an average molecular mass ranging between 1 kDa and 200 kDa, or even higher. Prior to crosslinking, polylysine may have an average molecular mass ranging between 1 kDa and 200 kDa, or even higher. Prior to crosslinking, PAA may have an average molecular mass ranging between 1 kDa and 200 kDa, or even higher. Prior to crosslinking, PDDA may have an average molecular mass ranging from 1 kDa to 1000 kDa, or even higher.

In certain embodiments, the crosslinked polymer is a glutaraldehyde crosslinked chitosan, chitosan derivative, PEI, polylysine, PAA, PDDA, or PVA.

In certain embodiments, the crosslinked polymer is obtained or obtainable by reacting a polymer, such as chitosan, a linear or branched PEI, polylysine, PAA, PDDA or PVA, and a suitable crosslinking agent, as known in the art, such as glutaraldehyde, particularly at a polymer to crosslinking agent ratio of 0.01 to 20 by weight, more particularly a polymer to crosslinking agent ratio of 0.05 to 15 or 0.1 to 12 by weight, or at a polymer to crosslinking agent molar ratio of about 0.005 to 50, such as at a polymer to crosslinking agent molar ratio of about 0.05 to 30 or 0.05 to 25. In particular embodiments, the reaction between the polymer and the crosslinking agent proceeds until completion of the reaction, i.e. until the concentration of at least the crosslinking agent no longer changes over time. For instance, the reaction between the polymer and the crosslinking agent may proceed for at least 30 min, for at least 1 h, for at least 2 h, for at least 5 h, at least 10 h or at least 15 h. The progress of the crosslinking reaction may be monitored by spectroscopy, such as UV-VIS spectroscopy.

In certain embodiments, a crosslinked chitosan is obtained or obtainable by reacting a chitosan and a suitable crosslinking agent, in particular glutaraldehyde, at a chitosan to crosslinking agent ratio ranging between 0.01 and 10 by weight, more particularly at a chitosan to crosslinking agent ratio ranging between 0.05 and 5 by weight or 0.05 to 2 by weight.

In certain embodiments, a crosslinked PEI is obtained or obtainable by reacting PEI and a suitable crosslinking agent, in particular glutaraldehyde, at a PEI to crosslinking agent ratio ranging between 0.1 and 20 by weight, more particularly at a PEI to crosslinking agent ratio ranging between 1 and 15 by weight or between 2 and 12 by weight.

In certain embodiments, the crosslinked polymer is obtained or obtainable by reacting a solution comprising a polymer and a solution of a suitable crosslinking agent, wherein the polymer concentration ranges from 0.05 wt% to 5 wt%, such as ranging from 0.1 wt% to 2.5 wt%, and wherein the concentration of the crosslinking agent ranges from 0.01 to 20 wt%, such as from 0.1 to 10 wt%.

The crosslinked nature of the polymer coating may be evaluated by scanning electron microscopy (SEM). It has been found that the crosslinked polymer provides a smooth coating on the electrode, compared to a non-coated electrode or an electrode coated with a non-crosslinked polymer. The degree of crosslinking in the crosslinked polymer may be determined by known methods. For instance, FTIR spectroscopy and Raman spectroscopy as known by the skilled person can be used to analyze the functional groups present in the crosslinked polymer. The degree of crosslinking can be estimated by examining the changes in the peak intensities or shifts in the characteristic absorption bands related to the functional groups involved in the crosslinking process. Analysis of the coating by X-ray Photoelectron Spectroscopy allows for the elemental identification and semi-quantitative analysis of the components of the coating. Other methods and techniques to assess the type of polymer making up the coating and its crosslinking include thermogravimetry analysis (TGA); determination of the CHN content by elemental analysis; determination of the sorption capacity of p-nitrophenol; differential scanning calorimetry; pyrolysis-gas chromatography mass spectrometry; and nuclear magnetic resonance spectroscopy (NMR).

In certain embodiments, the coating may further comprise conductive nanomaterials or nanocomposites to increase the electroactive surface area of the electrode and to attain an enhanced sensitivity and decrease in the limit of detection of the substance. Advantageously, a coating comprising a crosslinked polymer, such as a crosslinked chitosan polymer, mixed with a conductive nanomaterials, such as carbon nanotubes, was substantially more sensitive than the coating only comprising the crosslinked polymer. Suitable nanomaterials or nanocomposites comprise carbon nanotubes, graphene, fullerene, gold nanoparticles or nanorods, silver wires, palladium nanoparticles, zinc oxide nanoparticles, conductive polymers such as polyaniline, polypyrrole, poly(thiophene)s, poly(3,4-ethylenedioxythiophene), poly(p-phenylene sulfide), metal organic frameworks, or a combination of them with structural polymers such as polyethyleneimine, cellulose acetate, chitosan, polyvinyl chloride, polyvinyl butyral, polyvinyl acetate, polyurethane, polysterene, polyvinylpyrrolidone, polyvinyl alcohol or a combination of them, or a combination with surfactants such as sodium dodecyl sulfonate, sodium dodecylbenzenesulfonate, cetyltrimethylammonium chloride, cetyltrimethylammonium bromide, etc. The nanomaterials or nanocomposites as envisaged herein typically have at least one dimension (e.g. diameter) ranging between 0.5 nm and 500 nm, preferably between 1 nm and 250 nm. For instance, the carbon nanotubes as envisaged herein may have a diameter ranging between 0.5 nm and 120 nm, and a length ranging between 10 nm and 10µm.

The crosslinked polymer coating on at least the working electrode as envisaged herein may have a thickness ranging between 0.1 and 20 µm, particularly between 0.5 and 15 µm or between 1 and 10 µm, such as for instance determined by analyzing a scratched coating by scanning electron microscopy.

The sensor device or electrochemical sensor device may be a voltammetric and/or amperometric sensor device. An electrochemical sensor typically functions by the production of a current when a potential is applied between at least two electrodes, wherein the generated current is proportional to the concentration of the analyte of interest in the sample or bodily fluid. An amperometric sensor is a sensor that measures the current at a constant potential, and a voltammetric sensor is a sensor that measures current as a function of applied potential. As used herein, the term "amperometry" can refer to measuring the electric current between a pair of electrodes that are driving an electrolysis reaction, wherein the current is proportional to the concentration of an analyte. As used herein, the term "voltammetry" refers to techniques in which the relation between current and voltage is observed during an electrochemical process, particularly wherein the potential is varied as a function of time. As used herein, the term "voltammogram" refers to a graph of current vs. potential. For example, cyclic voltammetry (CV) sweeps the potential of linearly across a voltage range. Square-wave voltammetry (SWV) uses a square wave superimposed over a staircase function to provide a sweeping measurement that provides two sampling instances per potential. Advantageously, as a result of this sampling technique, the contribution to the total current that results from non-faradic currents is minimized. Like CV, the current is plotted as a function of potential.

The above indicated advantages provided by the crosslinked polymer coating make the sensor device according to the present application particularly suitable for measuring a substance in a bodily fluid, which comprises multiple biomolecules, such as proteins or lipids, that may otherwise adsorb on the electrode, thereby blocking the electrode surface for the detection of the substance.

In particular embodiments, the sensor device according to the present application is a point-of-care sensor device, i.e. a portable diagnostic device designed and adapted to provide on site testing of biological samples, in particular a bodily fluid. In certain embodiments, the bodily fluid is blood, plasma, urine or saliva. A point-of-care sensor device according to the present application comprises one or more electrodes, wherein said one or more electrodes comprise at least a working electrode, and a reference electrode and/or a counter electrode, wherein the surface of at least the working electrode is coated with a coating, wherein the coating comprises a crosslinked polymer as defined elsewhere herein. Advantageously, a point-of-care sensor according to the present application allows to analyze and monitor an analyte of interest in a simple and efficient way, close to the patient, such as in ambulatory settings, with reduced sample transportation and laboratory processing time, thus enabling faster diagnosis and treatment decisions. It has a high sensitivity and remarkable stability, even in the presence of possible interfering compounds, and is able to quantify and detect fluctuations in the concentration of the analyte of interest. A point-of-care sensor device according to the present application may be a voltammetric or an amperometric sensor device.

In certain embodiments, the point-of-care sensor device further comprises one or more of,
(i) a potentiostat, coupled to the one or more electrodes via a conductive connector. The potentiostat measures a voltage potential between the working electrode and the reference electrode of the point-of-care sensor device;
(ii) a processing unit, coupled to the potentiostat, for measuring electric signals via the electrodes and analyzing the electric signals, such as via suitable algorithms and software;
(iii) a memory unit, coupled to the processing unit, for storing data regarding the measured electric signals and/or the analysis thereof, and comprising instructions and algorithms for the processing unit. More in particular, the instructions for the processing unit may include the steps of (a) applying a specific voltage, e.g. a scanning/sweeping voltage in different ramp forms, such as linear sweep, a square-wave form, a differential-pulse form, a triangular wave form, to the point-of-care sensor, (b) measuring an electrical current in response to the applied voltage and/or generating at least one voltammogram, and (c) determining the presence, absence, or concentration of the substance in the sample, particularly bodily fluid of the subject using the measured current or the at least one voltammogram; and/or
(iv) a transmitter, coupled to the processing unit, particularly a wireless transmitter, for transmitting a signal or data to an external device, system or network, for instance wherein said external device, system or network includes a display or a user control for the sensor device. In some embodiments, the transmitter may be configured to transmit a signal based on the presence, absence or concentration of the analyte or substance to an external device, system or network. The transmitter may also be configured to transmit a signal or data periodically to the external device, system or network. Said external device, system, or network may include one or more of a smart phone, tablet computer, personal computer, network computer, or network server and the like. In certain embodiments, the external device may store and execute software applications and algorithms for processing the signals obtained by the sensor device.

In further particular embodiments, the sensor device as envisaged herein may be adapted to measure a substance in the dermal interstitial fluid. In particular embodiments, the sensor device as envisaged herein may be a microneedle-based sensor device. Advantageously, microneedle-based sensors are able to pierce the skin and allow to analyze and monitor analytes in interstitial fluid in a simple way, with a minimal invasiveness, particularly compared to a blood analysis. The microneedle-based sensor device as further described herein has a high sensitivity, even in the presence of possible interfering compounds, and is able to detect fluctuations in the concentration of the analyte of interest. It exhibits a remarkable stability and an outstanding resistance to biofouling. These properties make the microneedle-based sensor device particularly suitable as a wearable sensor device. For instance, the microneedle-based sensor device according to the present application may be a wearable patch. Said microneedle-based sensor device may be a voltammetric or an amperometric sensor device.

Accordingly, the present application further provides a microneedle-based sensor device, particularly suitable for measuring a substance in the dermal interstitial fluid, wherein the sensor device comprises:
- a substrate configured for attachment to the skin of the subject, such as via an adhesive; and
- a microneedle array comprising a plurality of microneedles, coupled to the substrate, wherein said plurality of microneedles comprises a microneedle working electrode made up of a first microneedle or a first series of microneedles, said microneedle(s) comprising a conductive material, and further comprising a microneedle reference electrode made up of a second microneedle or a second series of microneedles, said microneedle(s) comprising a conductive material, and/or a microneedle counter electrode made up of a third microneedle or a third series of microneedles, said microneedle(s) comprising a conductive material, wherein at least the microneedle working electrode is coated with the coating comprising the crosslinked polymer as envisaged herein, particularly a crosslinked polymer carrying a positively or negatively charged ionizable functional group, more particularly is coated with crosslinked chitosan, crosslinked chitosan derivative or a crosslinked polyethyleneimine, a crosslinked polyvinyl alcohol, or a mix or copolymer thereof.

The substrate is particularly an electrically insulative material, such as a plastic material (e.g., polyethylene terephthalate (PET), polyurethane (PU), polyethylene terephthalate glycol (PETG), and the like). The substrate may be flexible and/or bendable and/or stretchable. The substrate may further include an adhesive on at least one side of the substrate to enable secure attachment of the sensor device to the skin of the subject.

Microneedle arrays and methods for their production are known in the art. They typically comprise a plurality of microneedles. As used herein, the term "microneedle" generally refers to an elongated microstructure, extending between a base and top, such as a needle-like or pillar-like microstructure, with a geometry, length and girth (e.g., aspect ratio) adapted for piercing or insertion into the skin of a subject to provide physical access to the dermal interstitium (i.e. the liquid filled space between skin cells).

In microneedle arrays, the microneedle structures are typically (but not exclusively) orientated perpendicularly to the substrate, and are adapted to contact and pierce the skin of a subject, particularly when the sensor device is attached to the skin of a subject. The shape of the microneedle array, in particular its contact surface, i.e. the surface of the microneedle array adapted to contact the skin of the subject, is not particularly limiting and may be triangular, square, circular, or polygonal. The dimensions of the microneedle array, in particular its contact surface, may range between 5 and 20 mm. In addition, the shape and dimensions of each microneedle structure in the microneedle array is not particularly limiting. For instance, each microneedle structure may have a triangular, square, circular or polygonal shaped cross section, and may have a straight or tapered wall. The height of each microneedle (from base to top) may range between 500 µm and 2000 µm, such as between 600 µm and 1500 µm. The width or diameter at the base of each microneedle may range between 50 µm and 1000 µm. The microneedle structures can be spaced at various spacings and arrangements, which can be selected based on the application of the microneedle sensor device. Methods to deposit the crosslinked polymeric coating on the microneedle electrodes are known in the art.

In certain embodiments, said plurality of microneedles may comprise a plurality of conductive solid microneedles, for instance made of a metal, such as gold, platinum, silver, copper, and the like for the working or counter electrode, and silver, silver chloride or combinations thereof for the reference electrode. In certain embodiments, said plurality of microneedles comprises hollow microneedles, i.e. a plurality of microneedles with a hollow center, wherein the hollow center comprises a conductive material, such as a paste made of a conductive material for the working and counter electrode, and/or a silver/silver chloride paste for the reference electrode. The conductive material or paste thereof is not particularly limiting. It may be any conductive material known in the art, such as graphite or graphite paste, gold or platinum, or a paste comprising conductive nanomaterials, such as carbon nanotubes, graphene, gold nanoparticles or gold nanorods, or a combination thereof. The conductive material may be adjusted to the substance to be measured.

In certain embodiments, the microneedle-based sensor device further comprises one or more of, preferably integrated in and/or coupled to the substrate:
(i) a potentiostat, coupled to the one or more electrodes via a conductive connector. The potentiostat measures a voltage potential between the working electrode and the reference electrode of the microneedle array;
(ii) a processing unit, coupled to the potentiostat, for measuring electric signals via the electrodes and analyzing the electric signals, such as via suitable algorithms and software;
(iii) a memory unit, coupled to the processing unit, for storing data regarding the measured electric signals and/or the analysis thereof, and comprising instructions and algorithms for the processing unit. More in particular, the instructions for the processing unit may include the steps of (a) applying a specific voltage, e.g. a scanning/sweeping voltage in different ramp forms, such as linear sweep, a square-wave form, a differential-pulse form, a triangular wave form, to the microneedle array, (b) measuring an electrical current in response to the applied voltage and/or generating at least one voltammogram, and (c) determining the presence, absence, or concentration of the substance in the dermal interstitial fluid of the subject using the measured current or the at least one voltammogram;
(iv) a transmitter, coupled to the processing unit, particularly a wireless transmitter, for transmitting a signal or data to an external device, system or network, for instance wherein said external device, system or network includes a display or a user control for the sensor device. In some embodiments, the transmitter may be configured to transmit a signal based on the presence, absence or concentration of the analyte or substance to an external device, system or network. The transmitter may also be configured to transmit a signal or data periodically to the external device, system or network. Said external device, system, or network may include one or more of a smart phone, tablet computer, personal computer, network computer, or network server and the like. In certain embodiments, the external device may store and execute software applications and algorithms for processing the signals obtained by the sensor device;
(v) a plug-in connector as an interface between a disposable microneedle array and an electronics unit; and/or
(vi) means for the sustained attachment and/or proper insertion of the sensor device to the skin of the subject, such as e.g. a medical tape or a physiologically acceptable adhesive.

In some embodiments, the measured concentration of the substance may be time-dependent. Accordingly, in some embodiments, the microneedle-based sensor device is configured to be attached to the skin of a subject for about 1 day to about 30 days or even longer, advantageously without substantial biofouling, i.e. without substantial loss of the functionality or accuracy of the sensor device.

In the context of the present application, the substance is not particularly limiting and may be any analyte of interest that can be electrochemically detected, i.e. is able to exchange electrons with the working electrode in an oxidation or reduction process. The substance or analyte typically refers to a compound in a fluid, such as a bodily fluid, particularly in the interstitial fluid, wherein the presence and/or the concentration of the substance can be of interest to the subject or to a medical staff, such as to a medical doctor.

In particular embodiments, the substance is a small molecule, i.e. with a molecular weight between 0 and 3 kDa, such as a pharmaceutical compound or a metabolite thereof. The pharmaceutical compound may be an anti-cancer drug or a metabolite thereof, an opioid or metabolite thereof, an antibiotic or a metabolite thereof, an immunosuppressant or a metabolite thereof, an anxiolytic or metabolite thereof, an anticonvulsant or metabolite thereof, a hormone or neurotransmitter. Non-limiting examples include methotrexate or doxorubicin, leucovorin, amikacin, kanamycin, gentamicin, vancomycin, tobramycin, cyclosporin, procainamide, phenobarbital, phenytoin, esketamine, primidone, trazodone, carbamazepine, digoxin, quinidine, lidocaine, morphine, fentanyl, acetaminophen, naproxen, propofol, levodopa, mycophenolate, and the like.

The sensor device according to the present invention, particularly microneedle-based sensor device according to the present application allows to assess the presence, absence or concentration of the substance in a bodily fluid, particularly the interstitial fluid, over time. Advantageously, the sensor device according to the present application may be combined with a suitable drug delivery device adapted to release a drug or other substance in response to the measured presence, absence, or concentration of the substance. For instance, the microneedle-based sensor according to the present application may be interfaced with a closed-loop feedback drug-delivery system that uses the estimated substance (drug/metabolite) concentration determined by the sensor device according to the present invention, such as a microneedle-based sensor for controlling the timing and dose of a drug through subcutaneous delivery.

Accordingly, a further related aspect of the present application provides a system, particularly a closed loop system for the monitoring of a first substance and delivery of a second substance, particularly in the dermal interstitial fluid of a subject, wherein optionally said first and second substance are identical, the system comprising a sensor device according to the present application and a delivery device, such as a iontophoretic drug delivery device. More in particular, the system or closed loop system comprises:
- a sensor device, particularly a microneedle based sensor device, according to the present application, for electrochemically measuring a substance according to the present application, wherein said substance is the first substance;
- a first and second electrode, such as wherein the first electrode is the cathode and the second electrode is the anode;
- a drug delivery device comprising the second substance, operably connected to the first electrode and configured to release the second substance into the dermal interstitial fluid of the subject upon applying an electrical current between the first electrode and the second electrode; and
- a power supply connected to the first and second electrode.

In certain embodiments, the system, particularly closed loop system, further comprises one or more of:
- a processing unit coupled to the sensor device and the power supply, comprising instructions for controlling the monitoring of the first substance or a metabolite thereof in the dermal interstitial fluid and for controlling the administration of the second substance from the drug delivery device, particularly wherein the processing unit executes a dose-automation algorithm, such as wherein a signal generated by the sensor device is correlated to the absence, presence or concentration of the first substance in the dermal interstitial fluid, wherein this concentration is compared to a threshold value, and wherein the power supply is activated if the concentration of the first substance is below the threshold value;
- a memory unit, coupled to the processing unit, for storing data regarding the measured signals and/or the analysis thereof, and/or comprising instructions for the processing unit, such as a dose-automation algorithm;
- a transmitter, coupled to the processing unit, for transmitting a signal to an external device, system or network; and/or
- means for the sustained attachment and/or proper insertion of the system to the skin of the subject.

A further related aspect of the present application provides the use of a crosslinked polymer, particularly a crosslinked polymer carrying a positively or negatively charged ionizable functional group, for coating the surface of at least a working electrode of a sensor device for electrochemically measuring a substance in a bodily fluid, more particularly wherein said crosslinked polymer is crosslinked chitosan, crosslinked chitosan derivative, crosslinked polyethyleneimine, crosslinked polylysine, crosslinked polyallyl amine, crosslinked poly(diallyl dimethyl ammonium chloride), crosslinked polyvinylalcohol or a mix or copolymer thereof, optionally wherein the coating further comprises conductive nanomaterials or nanocomposites.

The sensor device, particularly a microneedle-based sensor device or a point-of-care sensor according to the present invention may be particularly used in the treatment of a disease or disorder, including but not limited to the monitoring of the therapeutic treatment. Following diagnosis of a subject with a physiological disorder or disease, a therapeutic treatment is prescribed, typically comprising the administration of a pharmaceutical compound. The sensor device, particularly a microneedle-based sensor device or a point-of-care sensor according to the present invention is particularly suited to monitor the presence, absence or concentration of the pharmaceutical compound or a metabolite thereof. This is particularly useful for medications where there is a clinical need to verify compliance and/or for drugs that have very strict dosage requirements and high toxicity. Advantageously, the microneedle-based sensor device according to the present invention may be configured to be attached to the skin of a subject for long periods of time without substantial biofouling, allowing for the sustained and continuous follow up of the presence, absence or concentration of the pharmaceutical compound or a metabolite thereof. This monitoring may allow for dosage regulation and/or monitoring of prescription compliance, thus ensuring a proper treatment of the disorder or disease.

A further related aspect provides a method for measuring a substance in a bodily fluid of a subject, particularly the dermal interstitial fluid of a subject, in particular wherein the substance is a pharmaceutical compound or a metabolite thereof, comprising the steps of:
(a) attaching a sensor device or a system according to the present application to the skin of the subject, thereby contacting the microneedle array with the substance in the bodily fluid of the subject; and
(b) measuring an electrical signal via the one or more electrodes, wherein the measured electrical signal is dependent on the presence, absence or concentration of the substance in the bodily fluid. In particular embodiments, step (b) includes (i) applying a voltage to the microneedle array, (ii) measuring an electrical current, particularly between the microneedle working electrode and microneedle counter electrode, in response to the applied voltage, and (iii) determining the presence, absence or concentration of the substance in the bodily fluid of the subject using the measured current or total charge.

The step (b) of applying a voltage to the microneedle array may comprise applying a voltage through a voltage window from -3.0 V to 3.0 V, particularly from -2.0 V to 2.0 V or from -1.0 V to 2.0 V, more particularly from -0.9 V to 1.6 V or from -0.7 V to 1.5 V. The applied voltage may be a linear voltage sweep, a square-wave form, a differential-pulse form, a triangular wave form.

In particular embodiments, the sensor device or the system according to the present application is continuously attached to the skin of the subject for a time period of a plurality of days and wherein step (b) is performed repeatedly during said time period, for determining the evolution of the presence, absence or concentration of the substance over time. For instance, the steps of applying a voltage and measuring a current may be repeated at an interval of one or more minutes to several hours, such as at an interval of about 1 min, 5 min, 10 min, 30 min, 1 h, 2 h, 3 h, 4 h, 5 h, 6 h, or even at longer intervals. Advantageously, the sensor device, particularly microneedle-based sensor device according to the present application, allows for the real time monitoring of *in vivo* drug concentrations, which may be critical for determining dosing and therapeutic adjustments.

In certain embodiments, step (b) may further comprise the step (iv) determining if the measured concentration exceeds a threshold value for the substance. In certain embodiments, the measured concentration of the substance generates a signal indicating the need to administer a pharmaceutical substance. In particular embodiments, the administering of a pharmaceutical substance, may be performed by iontophoresis. More in particular, when administering by iontophoresis, step (a) comprises attaching a system according to the present application to the skin of the subject, wherein the system comprises a microneedle sensor according to the present application, a first and a second electrode and a drug delivery device, wherein the drug delivery device of the system comprises a second substance and the method further comprises the step of (c) providing the second substance to the subject when the measured signal indicates the absence or a too low concentration of the first substance in the subject, particularly by applying a voltage between the first and second electrode, thereby actuating the drug delivery device.

### Examples

### Example 1. Fabrication of a microneedle patch for sensing

An electrochemical sensor based on microneedles (hereinafter abbreviated as MNs) was prepared as follows.

A/ A hollow MN array, hereinafter abbreviated as HMA, was selected as the wearable platform due to their ability to pierce the skin and reach the interstitial fluid (hereinafter abbreviated as ISF). The hollow MN arrays were designed using a computer-aided design (CAD) software. The arrays consist of 5 × 5 hollow MNs that have a designed pyramidal shape with 1 mm height, 0.75 mm width, 2 mm tip-to-tip interspacing, and 100 µm hole diameter. The hollow MN arrays were fabricated from polyether ether ketone (PEEK) sheets which were machined using a nanosecond-pulse, diode-pumped, solid-state laser operating at a wavelength of 355 nm. The drilling of the holes was done at the same time using the same laser system.

B/ The MN sensing patch was fabricated by filling the holes with the appropriate conductive paste to form a three-electrode electrochemical cell. Thus, the MN sensor incorporates a working electrode (WE) and counter electrode (CE) based on graphite paste, and a reference electrode (RE) based on silver/silver chloride (Ag/AgCI) paste. Once the hole of each MN is filled by using a spatula, the excess of the corresponding paste was carefully removed from the hole using a scalpel following the angle of the MN. Thereafter, conductive pastes were cured at 80 °C for 30 min in an oven.

This strategy of filling in the holes of the MNs with conductive inks enables the realization of an electrode that is protected with the MN body. This avoids damage to the electrode during the mechanical insertion of the MN-based electrode in the skin. The WE consists of 12 MNs, the RE consists of five MNs and the CE consists of eight MNs. The distribution of the electrodes followed the same configuration used in regular screen-printed electrodes, with a central WE and a CE and RE surrounding the WE to complete the electrochemical cell (Figure 1A).

Once the holes are filled with the corresponding conductive paste and cured, the MN sensor is attached to a planar connector for an easy connection to the potentiostat (Figure 1B). The termination of the connector follows the same pattern as a widely-used screen-printed electrode which enables a straightforward connection to the potentiostat while minimizing electrical noise. The MN sensor is stacked on top of the connector using double-side adhesive transfer tape and silver conductive paste to assure a proper electrical connection. Glue is used to isolate the edges of the MN sensor and avoid the solution to leak into the connections.

C/ The working electrode was subsequently functionalized with chitosan crosslinked with glutaraldehyde. To this end, first, a chitosan stock solution comprising 1.5 wt% chitosan in 2 wt% acetic acid was prepared. The chitosan used was obtained from shrimp shells, and is ≥75% deacetylated. Next, a crosslinked chitosan solution was prepared by dilution of the chitosan stock solution to a 0.15wt% chitosan aqueous solution, which was subsequently mixed with a glutaraldehyde (GA) solution to obtain a 1.5% GA solution. The reaction was allowed to proceed overnight. The reaction can be followed spectroscopically (UV-VIS). The crosslinked chitosan solution was thus obtained from a solution with a chitosan to GA ratio of 0.1. The crosslinked chitosan was deposited on the MNs WE (70 µL) and let dry in the oven at 40 °C for 30 min. The completed MN sensor (Figure 1C) can be stored until its use. As a comparative example, a non-crosslinked chitosan coating was applied to the MN WE of a second HMA. Other coatings are discussed in Example 3 below.

In addition, the crosslinked chitosan solution was deposited on screen-printed electrodes (SPEs). The electrochemical characterization of the coated SPEs is discussed in example 2B.

### Example 2. Characterization of the microneedle sensor

The properties and functional characteristics of the hollow microneedle array of example 1 were determined.

### A/ Morphology of the coated sensor

Scanning electron microscopy and optical microscopy were employed to assess the dimensions of the HMA and the modification of the HMA toward a functionalized MN sensor via the crosslinked chitosan (CHI/GA) coating. The MNs exhibited a height of 994 ± 56 µm and a width/diameter of 736 ± 12 µm with a hole diameter of 94 ± 7 µm.

The functionalization of the WE with the crosslinked chitosan (CHI/GA) solution provides anti-biofouling and adsorption capabilities to the WE allowing the continuous monitoring of methotrexate (MTX) in biofluids, as discussed in more detail in the following sections. The morphology of the coated electrodes was studied by scanning electron microscopy (SEM). The results are shown in Figure 2.

First, regular screen-printed electrodes (SPE) were employed as a model electrode to study the morphology under SEM of the deposited chitosan layer with and without crosslinker. The planar structure of the SPE allows a simpler characterization of the coatings. Figure 2A depicts the surface of a bare graphite electrode in the SPE showing its wrinkle structure with graphite flakes. Figure 2B shows the surface of the graphite electrode with the deposited chitosan layer without crosslinking (CHI layer/coating). The SEM images display similar shapes as per the bare graphite electrode meaning that the non-crosslinked chitosan coating remained highly porous on the electrode. In contrast, the crosslinked chitosan coating (or CHI/GA coating) exhibited a very smoothed layer on top of the graphite proving the crosslinking activity on the coating (Figure 2C).

Similar observations were made when considering the morphology and distribution of the crosslinked CHI/GA coating on the MN-based electrodes (via SEM). The RE displayed silver particles embedded in the polymeric paste (not shown). The CE electrode exhibited a morphology similar to the graphite bare electrode. Figure 2D and 2E display the crosslinked CHI/GA deposited on the MN WE. The images show the formation of a smooth coating on the electrodes. By analyzing a MN sensor with a scratched crosslinked CHI/GA layer, the thickness of the coating layer could be determined. The crosslinked coating at the base of the MN sensor exhibited a thickness of 9.0 ± 0.6 µm (N=3), while a thickness of 4.8 ± 0.1 µm (N=3) at the MN body was measured. It is suggested that the thickness at the body is less than at the base as a higher amount of solution is accumulated at the base of the MN during the evaporation of the solvent.

Attenuated total reflectance coupled with Fourier transform infrared (ATR-FTIR) was utilized for the chemical characterization of the crosslinked chitosan layer. For each spectrum, 128 scans were accumulated at 4 cm⁻¹ spectral resolution, the wavenumber range was between 4000 and 475 cm⁻¹. The spectra have been baseline-corrected, no additional treatments have been applied. The corrections of the spectra have been executed using the OPUS 8.2 software. The membranes were deposited on carbon-based electrodes in screen-printed electrodes to provide a planar surface that can be analyzed under the ATR module. Figure 3 shows the FTIR spectra of a non-crosslinked chitosan (CHI) and a crosslinked chitosan (CHI/GA) coating confirming the reaction of the amino groups of chitosan with GA. Both the NH₂ stretching bands at 3349 and 3283 cm⁻¹, which are observed in the CHI spectrum, could not be observed in the spectrum of the crosslinked coating. Moreover, a characteristic peak at 1150 cm⁻¹ associated with C-O-C bridges was shifted to a prominent 1110 cm⁻¹ peak.

### B/ Synergic effect of the crosslinked chitosan coating for methotrexate sensing.

The use of a crosslinked chitosan (CHI/GA) coating on an electrode produced a synergic effect for the detection of methotrexate (MTX): (i) the coating acts as an anti-biofouling layer, thus small molecules such as MTX diffuse toward the electrode's surface while blocking the entrance of macromolecules such as protein which is the main cause of biofouling; (ii) it allows the concentration/adsorption of MTX at the surface of the electrode causing an increment in the sensitivity; and (iii) a decrease in the oxidation potential. To prove this assumption, SPEs were modified with a non-crosslinked chitosan (CHI) and crosslinked chitosan (CHI/GA) coating to evaluate the synergic effect in PBS and PBS with albumin.

Figures 4A-4F depict the electrochemical oxidation of MTX on bare, non-crosslinked chitosan (CHI) and crosslinked chitosan (CHI/GA) coated SPEs in PBS after 10 min. The anodic signal was dramatically increased in the oxidation of 100 µM and 300 µM MTX in PBS pH 7.4 when using the crosslinked CHI-GA coating, exhibiting an increment in peak current (Iₚ) of 4.2 ± 0.2 fold and 3.0 ± 0.2 fold in comparison to the bare and CHI-coated SPEs, respectively. Importantly, the CHI/GA-coated SPE displayed a 7.9 ± 1.6 fold and 2.85 ± 1.0 fold increase in the Iₚ when the electrodes were interrogated under PBS with BSA (Figures 4D-4F). This suggests that while the non-crosslinked chitosan layer already provides some anti-biofouling capability to the sensor, crosslinking of the chitosan layer additionally provides for a highly sensitive detection, presumably due to the MTX adsorption in the coating. Moreover, the oxidation potential of the SPEs in BSA-enriched solution decreased from 0.92 V at bare SPE (uncoated) to 0.85 V for chitosan (CHI) coated SPE and 0.78 V for the crosslinked chitosan (CHI/GA) coated SPE. Interestingly, the CHI/GA coating unraveled a two-oxidation phenomenon at the surface of the WE.

The adsorption hypothesis was verified by executing a scan rate study to evaluate the mass transport behavior at the crosslinked chitosan coated CHI/GA electrode, by measuring cyclic voltammograms of 500 µM at different scan rates (i.e. 0.025 - 0.75 V s⁻¹). By plotting the anodic contribution against the scan rate, a linear relationship was obtained corresponding to an adsorption-controlled process. Furthermore, plotting the Iₚ against the square root of the scan rate showed a non-linear behavior. In addition, plotting the logarithm of the Iₚ against the logarithm of the scan rate showed a linear relationship with a slope of 0.76 which is higher than the theoretical value of the diffusion-controlled process (i.e. 0.5). Hence, the crosslinked chitosan coating endows the MTX sensor with an adsorption-controlled behavior. The influence of the adsorption time on the electrochemical response was assessed on a modified MN sensor (Figure 5). An absorption time of ten minutes was selected for further experiments as a compromise between time and sensitivity. The oxidation peak at the lowest potential was always selected as the main analytical signal.

### C/ In vitro analytical characterization

The analytical performance of the chemical sensor prepared in example 1 was assessed by using square-wave voltammetry (SWV). SWV curves were obtained by dropping 150 µL of phosphate buffer saline (PBS, pH 7.4) at different concentrations of methotrexate (MTX). The SWV parameters that were used: potential range of 0.4-1.1 V, 10 Hz frequency, 25 mV amplitude and 5 mV step potential. Peak current (Iₚ) was used to construct the calibration curve. To evaluate the biofouling effect on the MN sensor, PBS with 20.6 g L⁻¹ of bovine serum albumin (BSA) was used to emulate the total amount of protein found in ISF.
(i) a reference MN electrochemical sensor without was first the crosslinked chitosan (CHI/GA) coating analytically characterized. The main results are represented in Figure 6. The specific solution is dropped on the MN sensor to perform the electrochemical interrogation. SWVs are measured for increasing concentrations of methotrexate (MTX) in PBS pH 7.4 (triplicate per each concentration) and a calibration curve was determined (Figure 6A). The linear range expanded from 25 µM to 300 µM which fits with the MTX levels during chemotherapy. The slope of the calibration curve was 3.3 nA µM⁻¹ with a relative standard deviation (RSD) of 5.0 ± 0.9 % in each of the concentrations (25 µM to 300 µM) and a limit of detection (LOD) of 8.8 µM. As the MN sensor aims for continuous operation, the repeatability of the electrochemical signal is paramount. A repeatability study was setup by determining the SWV with 25 µM, 100 µM (Figure 6B) and 200 µM, respectively. The tests exhibited an outstanding performance for 10 repetitions of RSD_{25 µM} = 9.8%, RSD_{50 µM} = 5.9%, and RSD_{200 µM} = 2.5%. As other electroactive molecules can be present in the biofluids, a selectivity test was executed with the main molecules found in serum and potentially in ISF (i.e. glycine, phenylalanine, tyrosine, uric acid, ascorbic acid) as well as some common drugs used in daily life (i.e. caffeine, and paracetamol) (Figure 6C). A RSD of 5% was obtained among all experiments, proving the proper analysis of MTX even with the presence of other interferents. These results demonstrate that the bare sensor exhibited excellent performance in buffer solutions. The next step was to evaluate the piercing effect on the analytical response. The test consisted of the insertion of the MN sensor on 10 layers of Parafilm and performing a calibration curve. The slopes slightly decrease from 3.1 nA µM⁻¹ (before insertion) to 2.8 nA µM⁻¹ (1^{st} post insertion) and 2.7 nA µM⁻¹ (2^{nd} post insertion). These results suggest that the major difference appears after the first insertion test, and thereafter, the analytical performance is stabilized. This effect can be due to the mechanical force endured on the conductive electrode during the piercing which can modify the exceeding conductive paste from out of the hole, thus decreasing the electroactive surface area. This effect can be mitigated in real applications by recording the calibration curve after performing an insertion test in the Parafilm. A porcine skin insertion test was also executed with the same MN sensor. In this case, a dramatic decrease of 1.6 fold on the slope after the porcine insertion (61% relative to the previous curve) was obtained worsening the analytical capabilities to detect MTX in ISF. Porcine skin has fatty components that might adsorb on the graphite paste on the working electrode, therefore, blocking the surface available for the oxidation of MTX. For this reason, the use of an anti-biofouling layer seems necessary for the analysis of MTX in biofluids.

As discussed above and represented in Figure 4, the deposition of a crosslinked chitosan (CHI/GA) layer proved to enhance the MTX detection in SPE. Therefore, the same coating was deposited on the MN sensors, and subsequently, the analytical performance was characterized (see also Figure 7). The crosslinked chitosan coating (CHI/GA) allows the adsorption of MTX into the layer close to the electrode's surface and prevents macromolecules such as proteins, the main cause of biofouling, to be adsorbed at the surface (Figure 7A). Thus, CHI/GA is suggested to have a synergic effect that is beneficial for continuous operation in biofluids. The analytical characterization of the crosslinked chitosan coated sensor was first evaluated in PBS pH 7.4 at room temperature (i.e. 21 C°) by interrogating the MN sensor at each concentration after 10 min adsorption time (see Figure 4C). Figure 7B displays the calibration curve exhibiting a slope of 3.6 nA µM⁻¹ within a dynamic range from 25 to 500 µM and a LOD of 10.0 µM which fits the therapeutic range of interest. As the crosslinked chitosan coated MN sensor aims to pierce the skin, the same f-MN sensor was assessed under 34 C° as average skin temperature. Figure 7C and 7D show the SWV upon increasing concentrations of MTX, and the corresponding calibration curve, respectively. At 34 °C, the slope substantially increased to 4.5 nA µM⁻¹ while maintaining a similar dynamic range and LOD (12.1 µM). It has been reported that temperature provides higher mass transfer in the Nernst layer as well as it has been empirically shown its effect on the increment in peak current and the shift toward more negative potentials employing SWV. The chitosan crosslinked coated MN sensor thus demonstrates analytical capabilities to be used at skin temperature. However, further experiments were performed at room temperature for easy handling of the setup. The selectivity test was executed by adding 100 µM of the interferent (or 200 µM for glycine) to 100 µM MTX before the electrochemical interrogation. Figure 7E shows the Iₚ obtaining a RSD of 7.8% between all measurements. Thus, the crosslinked chitosan coated MN sensor did not exhibit interferences on the MTX signal with regularly encountered electroactive molecules in biofluids. As one of the features of the crosslinked chitosan coating is the anti-biofouling capacity (see Figure 4F vs Figure 4C), PBS with BSA as biofouling agent was employed for the testing. SWVs of the f-MN sensor were measured in the BSA in solution and the corresponding calibration curve is illustrated in Figure 7F. A slope of 2.3 nA µM⁻¹ and LOD of 19.9 µM showed an effect of the protein on the analytical performance: the crosslinked chitosan coating thus allowed for MTX detection in the therapeutic range. The repeatability of the f-MN sensor was assessed for 25 µm, 100 µm and 300 µM in PBS-BSA. The results showed excellent repeatability (i.e. RSD_{25µM} = 4.8%, RSD_{100µM} = 5.5%, and RSD_{300µM} = 4.3%).

To emulate the ability of the crosslinked chitosan coated sensor (referred to herein also as f-MN sensor) for continuous monitoring of MTX, the f-MN sensor was interrogated under artificial interstitial fluid (AISF). Figure 8A depicts the SWVs of the f-MN sensor upon increasing concentrations of MTX. Figure 8B shows the calibration curve with a slope of 2.4 nA µM⁻¹, LOD of 10.2 µM within a linear range from 25 to 200 µM. It is important to mention that different f-MN sensors were used for the tests in buffer and artificial interstitial fluid (AISF), thus differences in the sensitivity of the systems can be obtained. As the manufacturing process is performed in-house, variations in the fabrication process can lead to different electrochemical performances. Subsequently, the f-MN sensor was interrogated under AISF with BSA (Figures 8C and 8D). The slope of the calibration curve of 1.8 nA µM⁻¹ and LOD of 12.3 µM showed a decrease in the analytical performance similar to the behavior studied using PBS and PBS-BSA solutions. Nevertheless, the f-MN sensor allowed the monitoring of MTX within the range of interest. An important feature of a transdermal device aiming for continuous monitoring is the ability to detect fluctuations in the target analyte. Hence, a reversibility test in AISF with BSA was attained during 8 cycles of increasing and decreasing concentrations of MTX (50 µM -100 µM - 200 µM cycle). Subsequently, a calibration curve was constructed by plotting the Iₚ gathered from the reversibility test, and a slope of 1.8 nA µM⁻¹ was obtained which is the same value as for the previous calibration curve before the reversibility test proving the stability of the f-MN sensor. Besides, excellent repeatability of each concentration was obtained (RSD_{50µM} = 10.9%, N=5; RSD_{100µM} = 7.5%, N=8; and RSD_{300µM} = 3.5%, N=4).

Long-term stability is essential for wearable devices that aim to continuously monitor targets once are inserted or applied to the body. Therefore, the evaluation of the performance of the f-MN sensor in the AISF enriched with BSA was further studied during long-term analysis. First, the stability of the electrochemical readout was assessed continuously every 10 min without any MTX for 16 h. This experiment provided information on the potential degradation of the CHI/GA layer and the rise of background peaks that could lead to false positives. Importantly, the SWVs of the blank did not display the emergence of a background peak. Thereafter, the f-MN sensor was analyzed under 50 µM MTX for 5 h exhibiting a repeatable peak current with a RSD of 11.1% (Figure 8E). The f-MN sensor was subsequently analyzed under 100 µM MTX for 13 h exhibiting a repeatable peak current with a RSD of 4.7% (Figure 8F). Finally, the f-MN sensor was interrogated with 200 µM MTX for 13 h exhibiting a RSD of 4.0% on the peak current (Figure 8G). Moreover, the SWVs exhibited a constant oxidation potential of the MTX signal between 0.72 V and 0.74 V. Finally, the average Iₚ obtained during the stability tests was used to construct a calibration plot (Figure 8H). The calibration curve exhibited a slope of 1.8 nA µM⁻¹ which is the same slope as the previous experiments proving the stability of the f-MN sensor after more than three days of operation (i.e. two days during the stability test and one day for the reversibility test). Overall, the f-MN sensor proved to be a highly stable sensor for the monitoring and detection of fluctuations of MTX in protein-enriched media.

### D/ Ex vivo analytical characterization of the MN sensing patch

The analytical performance of the MN sensing patch was assessed under a Franz diffusion cell configuration. Dermatomed neonatal porcine skin (500 µm thickness) was attached to the donor compartment. The donor compartment is filled with PBS solution and is subsequently spiked using the sampling port with the corresponding methotrexate (MTX) amount by using the standard addition method. The MN sensor that is previously pierced in the skin can monitor the concentration of MTX in the donor compartment. For the decreasing concentration curve, extraction of the MTX solution through the sampling port and the addition of fresh buffer was performed. The SWV measurements were launched every 10 min.

The ex *vivo* test is an important step in the characterization of MN-based devices. Franz diffusion cells are well-established setups used in ex *vivo* tests to emulate a real scenario while controlling the concentration of the analyte at the receptor compartment. Importantly, the setup mimics physiological conditions by employing porcine skin and a heating system. The concentration at the receptor compartment can be varied by spiking the analyte through the sampling port to increase the concentration at the inner side of the skin or the concentration can be diluted by the extraction of the solution and introducing fresh solution (without the analyte) to the Franz cell. Figure 9A illustrates the Franz diffusion cell setup where the crosslinked chitosan coated sensor (f-MN sensor) is situated in the donor compartment (previously pierced in porcine skin). Through the skin, the f-MN sensor can detect methotrexate (MTX) that is present in the solution in the donor compartment. Once the f-MN sensor was established on the Franz cell and connected to the potentiostat, a calibration curve was executed. Figure 9B displays the SWVs from the increasing concentrations of MTX in the receptor compartment. In real applications, the levels of a patient under MTX treatment expand from high concentration (mM range) due to the administration of a high-dose MTX to low µM levels which levels determine the toxicity to the body and a potential rescue with leucovorin. Therefore, the interest is to monitor the clearance from MTX to rapidly detect toxic levels of MTX meaning the monitoring from high concentration to low concentration in the ISF. Hence, the f-MN sensor was interrogated with SWV under decreasing concentrations of MTX ranging from 400 µM to 25 µM MTX in the Franz cell setup (Figure 9C). The calibration curves from the increasing and decreasing concentrations of MTX exhibited a slope of 1.75 nA µM⁻¹ and 1.76 nA µM⁻¹ with LODs of 22.5 µM and 9.3 µM, respectively (Figure 9D), proving the analytical capability of the f-MN sensor to monitor the decrease of MTX in the donor compartment through the skin. Overall, the f-MN sensor was able to monitor increasing, and importantly, decreasing concentrations of MTX through the skin which validates its potential use to monitor MTX clearance in patients.

### Example 3. Assessment of different coatings

A/ Coatings made up of different crosslinked polymers were prepared and assessed in a similar manner as in Examples 1C and 2B. All experiments were performed with screen-printed electrodes (SPEs) as the standard electrochemical platform. The crosslinking agent used in all experiments was glutaraldehyde. Time of adsorption was set at 10 min before launching the experiment. The following coatings were tested: crosslinked chitosan comprising carbon nanotubes, crosslinked polylysine, crosslinked polyallylamine (PAA) and crosslinked poly(diallyldimethylammonium chloride) (PDDA).
* Crosslinked chitosan with carbon nanotubes. Carbon nanotubes (CNTs) (2 mg/ml) were added and dispersed in the crosslinked chitosan solution prepared in example 1C. Carbon nanotubes are widely used to increase the electroactive surface area on the electrode. After depositing the dispersion of CNTs with CHI-GA on the electrode, a 1.9 enhancement in the electrical signal was obtained in comparison to the deposited CHI-GA (without CNTs) (Figure 10A).
* Crosslinked polylysine. Crosslinked and non-crosslinked polylysine were deposited on the surface of the electrode and the effect of crosslinking was subsequently assessed. To that end, several crosslinked PL solutions were prepared comprising 0.5 wt% PL and different glutaraldehyde concentrations (0.5wt%; 1.0wt%; 2.0wt% or 5.0wt%). Up to a 13.7-fold enhancement in the electrical signal was obtained by using the crosslinked polymer in comparison to the non-crosslinked polylysine (Figure 10B).
* Crosslinked poly(allylamine) (PAA). Crosslinked and non-crosslinked PAA were deposited on the surface of the electrode and the effect of crosslinking was subsequently assessed. To that end, a crosslinked PAA solution was prepared comprising 1.0 wt% PAA and 2.0wt% GA. A 10.3 fold enhancement in the electrical signal was obtained by using the crosslinked polymer in comparison to the non-crosslinked polymer (Figure 10C).
* Crosslinked poly(allyldimethylammonium chloride) (PDDA). Crosslinked and non-crosslinked PDDA were deposited on the surface of the electrode and the effect of crosslinking was subsequently assessed. To that end, a crosslinked PDDA solution was prepared comprising 1.0 wt% PDDA and 2.0wt% GA. A 5.3 fold enhancement in the electrical signal was obtained by using the crosslinked polymer in comparison to the non-crosslinked polymer (Figure 10D).

B/ The coating with a crosslinked polymer provides key functionalities to the sensor. Several crosslinked polymers were tested and optimized toward higher sensitivity of the sensor for the detection and monitoring of MTX.

More in particular and without wishing to be bound by theory, the crosslinking degree of the polymer, such as chitosan, can (i) have an effect on the exposure of more sides where MTX can be adsorbed, (ii) limit the hydration of the coating, and, thus, the free diffusion of analytes, and/or (iii) block the entrance of macromolecules (e.g. proteins).
* Crosslinked chitosan. The composition of the crosslinked chitosan was evaluated employing the SPE platform as a gold standard electrochemical platform (same procedure as in Example 2B). Several crosslinking degrees were tested by varying the ratio between the polymer and crosslinking agent (Table1). Under the conditions of the test, the highest increase in peak current was obtained with a coating composition with a polymer-to-crosslinking agent ratio of 0.1.

**Table 1. Effect of different crosslinked chitosan based coatings**

| Polymer | Polymer concentration (wt%) | Crosslinking agent (wt%) | Ratio polymer/crosslinking agent | Signal increase (-fold)* |
|---|---|---|---|---|
| Chitosan | 0.15% | 1.5% GA | 0.1 | 3.9 |
| Chitosan | 0.15% | 0.15% GA | 1 | 2.2 |
| Chitosan | 0.15% | 0.015% GA | 10 | 0.8 |
| Chitosan | 0.15% | 15% GA | 0.01 | 1.5 |
| Chitosan | 0.15% | 0.5% GA | 0.3 | 2.4 |
| Chitosan | 0.15% | 1% GA | 0.15 | 3.6 |
| Chitosan | 0.15% | 3% GA | 0.05 | 3.5 |
| Chitosan | 0.15% | 5% GA | 0.03 | 2.8 |

| | | | | |
|---|---|---|---|---|
| GA: glutaraldehyde *signal increase of the peak current (Iₚ) compared to the Ip of the bare electrode. * Crosslinked PEI. The same procedure was executed by using another polymer (i.e. PEI). PEI is well-known as a positively charged polymer with branched primary amine groups. The branched chemical groups can be crosslinked with a crosslinking agent (e.g. GA). The crosslinked polymer was deposited on the SPE platform (same procedure as in Example 2B) using different crosslinking degrees (Table 2). PEI is a hydrophilic polymer that solubilizes in aqueous solution. Therefore, the crosslinking of the polymer at the surface of the electrode also avoids the release of the polymer into the solution, and thus ensures a continuously enhanced signal generation. Under the conditions of the test, the highest increase in peak current was obtained with a coating composition with a polymer-to-crosslinking agent ratio of 10. | | | | |

**Table 2. Effect of different crosslinked PEI based coatings**

| Polymer | Polymer concentration | Crosslinking agent | Ratio polymer/crosslinking agent | Signal increase (-fold)* |
|---|---|---|---|---|
| | (wt%) | (wt%) | | |
| PEI | 1% | 1% GA | 1 | 2.0 |
| PEI | 1% | 10% GA | 0.1 | 0.3 |
| PEI | 1% | 0.1% GA | 10 | 4.7 |
| PEI | 2.5% | 0.25% GA | 10 | 4.8 |

| | | | | |
|---|---|---|---|---|
| GA: glutaraldehyde *signal increase of the peak current (Iₚ) compared to the Ip of the bare electrode. | | | | |

Providing a polymeric coating wherein the polymers are crosslinked on a sensor allows to obtain an enhanced electrochemical signal from the sensor. The crosslinking of the polymers provides the coating with improved features that can be applied for the sensing or monitoring of specific analytes.

### Conclusions

The above examples demonstrate MN-based electrochemical sensors for the transdermal monitoring of MTX. The functionalization of the MN sensor (f-MN sensor) with a crosslinked polymer coating, in particular a crosslinked chitosan coating conferred a synergic effect of anti-biofouling and preconcentration capabilities for the continuous operation of the f-MN sensor. First, a HMA was modified with conductive pastes to create a three-electrode electrochemical system in a single patch. Thereafter, the MN sensor was analytically characterized with and without the crosslinked chitosan coating to unravel its unique properties for the detection of MTX in protein-enrich artificial interstitial fluid (AISF). The full *in vitro* analysis exhibited remarkable results within the linear range of high-dose MTX administration levels in cancer patients. The reversibility and long-term stability of the readout exhibited an outstanding performance which is essential for wearable devices. The f-MN sensor was assessed under an *ex vivo* configuration in porcine skin proving the ability to transdermally monitor decreasing concentrations of MTX, an essential future to observe the MTX clearance in patients under MTX treatment.

## Claims

1. A sensor device for electrochemically measuring a substance in a sample, such as a bodily fluid, said sensor device comprising one or more electrodes, wherein said one or more electrodes comprise at least a working electrode, a reference electrode and/or a counter electrode, wherein the surface of at least the working electrode is coated with a coating, **characterized in that** the coating comprises a crosslinked polymer, preferably a crosslinked polymer carrying a positively or negatively charged ionizable functional group.

2. The sensor device according to claim 1, wherein the crosslinked polymer is a crosslinked chitosan, a crosslinked chitosan derivative, a crosslinked polyethyleneimine, a crosslinked polylysine, a crosslinked polyallyl amine, a crosslinked poly(diallyl dimethyl ammonium chloride), a crosslinked polyvinyl alcohol, or a mix or copolymer thereof.

3. The sensor device according to claim 1 or 2, wherein the crosslinked polymer has or is obtained by reacting a polymer and a crosslinking agent at a polymer to crosslinking agent ratio of 0.01 to 20 by weight, particularly a polymer to crosslinking agent ratio of 0.05 to 15 by weight.

4. The sensor device according to any one of claims 1 to 3 wherein the coating further comprises nanomaterials or nanocomposites to increase the electroactive surface area of the electrode and attain a decrease in the limit of detection of the substance.

5. The sensor device according to any one of claims 1 to 4, wherein the sensor device is an electrochemical point-of-care sensor device.

6. The sensor device according to any one of claims 1 to 4, particularly for measuring the substance in the dermal interstitial fluid of a subject, wherein the device comprises:
- a substrate configured for attachment to the skin of the subject; and
- a microneedle array comprising a plurality of microneedles, coupled to the substrate, wherein said plurality of microneedles comprises a microneedle working electrode made up of a first microneedle or a first series of microneedles comprising a conductive material, and further comprising a microneedle reference electrode made up of a second microneedle or a second series of microneedles comprising a conductive material, and/or a microneedle counter electrode made up of a third microneedle or a third series of microneedles comprising a conductive material, wherein at least the microneedle working electrode is coated with the coating comprising the crosslinked polymer, particularly a crosslinked polymer carrying a positively or negatively charged ionizable functional group, more particularly is coated with crosslinked chitosan, crosslinked chitosan derivative or a crosslinked polyethyleneimine, crosslinked polylysine, crosslinked polyallyl amine, crosslinked poly(diallyl dimethyl ammonium chloride), a crosslinked polyvinyl alcohol, or a mix or copolymer thereof.

7. The sensor device according to claim 6, wherein:
- said plurality of microneedles comprises a plurality of microneedles with a hollow center, wherein the hollow center comprises a conductive material, such as a paste made of a conductive material for the working and counter electrode, and/or a silver/silver chloride paste for the reference electrode; or
- said plurality of microneedles comprises a plurality of conductive solid microneedles.

8. The sensor device according to any one of claims 1 to 7, wherein the sensor device is a voltammetric or an amperometric sensor device.

9. The sensor device according to any one of claims 1 to 8, wherein the sensor device further comprises one or more of:
- a potentiostat, coupled to the one or more electrodes via a conductive connector;
- a processing unit, coupled to the potentiostat, for measuring electric signals via the electrodes and analysing the electric signals;
- a memory unit, coupled to the processing unit, for storing data regarding the measured electric signals and/or the analysis thereof, and comprising instructions for the processing unit;
- a transmitter, coupled to the processing unit, for transmitting a signal to an external device, system or network, for instance wherein said external device, system or network includes a display or a user control for the sensor device;
- optionally, a plug-in connector as an interface between a disposable microneedle array and the electronics unit; and/or
- optionally, means for the sustained attachment and/or proper insertion of the sensor device to the skin of the subject.

10. The sensor device according to claim 9, wherein the instructions for the processing unit include the steps of (i) applying a specific voltage or scanning/sweeping voltage in different ramp forms, such as linear sweep, a square-wave form, a differential-pulse form, a triangular wave form, to the microneedle array, (ii) measuring an electrical current in response to the applied voltage, and (iii) determining the concentration of the substance in the sample, such as a bodily fluid or the dermal interstitial fluid of the subject, using the measured current.

11. The sensor device according to any one of claims 1 to 10, wherein the substance is a small molecule, such as methotrexate, doxorubicin, leucovorin, amikacin, kanamycin, gentamicin, vancomycin, tobramycin, cyclosporin, procainamide, phenobarbital, phenytoin, esketamine, primidone, trazodone, carbamazepine, digoxin, quinidine, lidocaine, morphine, fentanyl, acetaminophen, naproxen, propofol, levodopa, or mycophenolate.

12. A system, particularly a closed loop system, for the monitoring of a first substance and delivery of a second substance in the dermal interstitial fluid of a subject, wherein optionally said first and second substance are identical, the system comprising:
- a sensor device for electrochemically measuring a substance according to any one of claims 6 to 11, wherein said substance is the first substance;
- a first and second electrode, particularly wherein the first electrode is the cathode and the second electrode is the anode;
- a drug delivery device comprising the second substance, wherein the drug delivery device is operably connected to the first electrode and is configured to release the second substance into the dermal interstitial fluid of the subject upon applying an electrical current between the first electrode and the second electrode; and
- a power supply connected to the first and second electrodes.

13. The system according to claim 12, further comprising one or more of:
- a processing unit coupled to the sensor device and the power supply, comprising instructions for controlling the monitoring of the first substance or a metabolite thereof in the dermal interstitial fluid and for controlling the administration of the second substance from the drug delivery device, particularly wherein the processing unit correlates a signal generated by the sensor device to the concentration of the first substance in the dermal interstitial fluid, compares this concentration to a threshold value, and activates the power supply if the concentration of the first substance is below the threshold value;
- a memory unit, coupled to the processing unit, for storing data regarding the measured signals and/or the analysis thereof, and/or comprising instructions for the processing unit;
- an algorithm for sensing and actuating that calculates the amount of the second substance that needs to be delivered according to the concentration in the interstitial fluid and is extrapolated to the current and time needed in the drug delivery device;
- a transmitter, coupled to the processing unit, for transmitting a signal to an external device, system or network;
- means for the sustained attachment and/or proper insertion of the system to the skin of the subject; and/or
- a data interface to follow the sensing and drug delivery in a user-friendly manner.

14. The sensor device according to any one of claims 1 to 11 or the system according to any one of claims 12 to 13, wherein said sensor device or said system is a wearable device.

15. Use of a crosslinked polymer, particularly a crosslinked polymer carrying a positively or negatively charged ionizable functional group, for coating the surface of at least a working electrode of a sensor device for electrochemically measuring a substance in a bodily fluid, more particularly wherein said crosslinked polymer is crosslinked chitosan, crosslinked chitosan derivative, crosslinked polyethyleneimine, crosslinked polylysine, crosslinked polyallyl amine, crosslinked poly(diallyl dimethyl ammonium chloride), crosslinked polyvinyl alcohol or a mix or copolymer thereof, optionally wherein the coating further comprises conductive nanomaterials or nanocomposites.
